# EUROPÄISCHE PATENTANMELDUNG

(11) **EP 4 011 861 A1**
(43) Veröffentlichungstag der Anmeldung: **15.06.2022**
(21) Anmeldenummer: 20213498.7
(22) Anmeldetag: 11.12.2020
(51) Int. Cl.: C07C 68/02, C07C 69/96, C08G 64/30

(54) **VERFAHREN ZUR HERSTELLUNG VON DIARYLCARBONATEN MIT GERINGEM PHENOLGEHALT IM ABWASSER**

(71) Anmelder: Covestro Deutschland AG, 51373 Leverkusen (DE)
(72) Erfinder: GARSKA, Bernd, 2040 Antwerpen (BE); HEIJL, Jan, 2040 Antwerpen (BE); SLUYTS, Erik, 2930 Brasschaat (BE); YAO, Xiaoxin, 201199 Shanghai (CN)
(74) Vertreter: Levpat

(57) **Zusammenfassung**

Gegenstand der vorliegenden Erfindung ist ein Verfahren zur Herstellung eines Diarylcarbonats durch Reaktion eines Monophenols oder mehrerer Monophenole mit Phosgen in einem inerten Lösungsmittel in Gegenwart von Alkali und einer Stickstoffbase im sogenannten Phasengrenzflächenverfahren. Das erfindungsgemäße Verfahren zeichnet sich dadurch aus, dass - verglichen mit dem Stand der Technik - der Gehalt an Monophenol im Abwasser deutlich verringert wird. Insbesondere Gegenstand der vorliegenden Erfindung ist ein solches Phasengrenzflächenverfahren zur Herstellung von Diphenylcarbonat. Weiterer Gegenstand der vorliegenden Erfindung ist auch ein Verfahren zur Herstellung von Polycarbonat umfassend das erfindungsgemäße Verfahren zur Herstellung eines Diarylcarbonats.

## Beschreibung

Gegenstand der vorliegenden Erfindung ist ein Verfahren zur Herstellung eines Diarylcarbonats durch Reaktion eines Monophenols oder mehrerer Monophenole mit Phosgen in einem inerten Lösungsmittel in Gegenwart von Alkali und einer Stickstoffbase im sogenannten Phasengrenzflächenverfahren. Das erfindungsgemäße Verfahren zeichnet sich dadurch aus, dass - verglichen mit dem Stand der Technik - der Gehalt an Monophenol im Abwasser deutlich verringert wird. Insbesondere Gegenstand der vorliegenden Erfindung ist ein solches Phasengrenzflächenverfahren zur Herstellung von Diphenylcarbonat. Weiterer Gegenstand der vorliegenden Erfindung ist auch ein Verfahren zur Herstellung von Polycarbonat umfassend das erfindungsgemäße Verfahren zur Herstellung eines Diarylcarbonats.

Die Herstellung von Diarylcarbonaten durch ein Phasengrenzflächenverfahren ist prinzipiell in der Literatur bekannt, siehe z.B. Chemistry and Physics of Polycarbonates, Polymer Reviews, H. Schnell, Vol. 9, John Wiley and Sons, Inc. (1964), S. 50/51. Im US-P 4 016 190 wird ein Herstellungsverfahren von Diarylcarbonaten beschrieben, das bei Temperaturen größer 65 °C betrieben wird und bei dem Stickstoffbasen als Katalysatoren eingesetzt werden.

Die Herstellung von Diarylcarbonaten hat wiederum eine große technische und wirtschaftliche Bedeutung in der Herstellung von Polycarbonaten nach dem sogenannten Schmelzeumesterungsverfahren.

Diarylcarbonate, die zur Herstellung von Polycarbonaten nach dem Schmelzeumesterungsverfahren eingesetzt werden können, sind insbesondere Di-C₆-C₁₄-Arylester, vorzugsweise die Diester von Phenol oder substituierten Phenolen, also Diphenylcarbonat oder z.B. Bissalicylcarbonat. Bezogen auf 1 Mol Diphenol werden die Diarylcarbonate in 1,01 bis 1,30 Mol, bevorzugt in 1,02 bis 1,15 Mol eingesetzt.

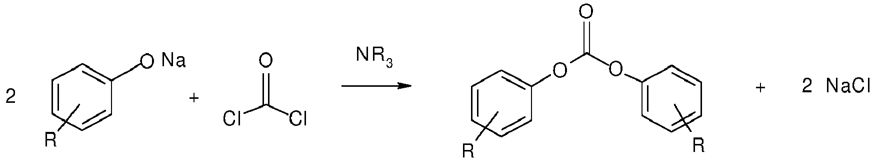

Das ausreagierte, höchstens noch Spuren an (kleiner 2 ppm) Chlorkohlensäurearylestern enthaltende mindestens zweiphasige Reaktionsgemisch lässt man zur Phasentrennung absitzen. Die wässrige alkalische Phase (Reaktionsabwasser) wird abgetrennt und die organische Phase mit verdünnter Salzsäure und Wasser extrahiert. Die vereinigten Wasserphasen werden der Abwasseraufarbeitung zugeführt, wo Lösungsmittel- und Katalysatoranteile durch Strippen oder Extraktion abgetrennt und rückgeführt werden. Anschließend können nach der Einstellung eines bestimmten pH-Wertes von z.B. von 6 bis 8, z.B. durch Salzsäurezugabe, die gegebenenfalls noch verbleibenden organischen Verunreinigungen wie z.B. Monophenol durch Behandlung mit Aktivkohle entfernt und die Wasserphasen der Chloralkalielektrolyse zugeführt werden.

In einer anderen Variante der Aufarbeitung wird das Reaktionsabwasser nicht mit den Waschphasen vereinigt, aber nach Strippen oder Extraktion zur Abtrennung von Lösungsmitteln und Katalysatorresten, auf einem bestimmten pH-Wert von z.B. 6 bis 8, z.B. durch Salzsäurezugabe eingestellt und nach Abtrennung der noch verbleibenden organischen Verunreinigungen wie z.B. Monophenol durch Behandlung mit Aktivkohle der Chloralkalielektrolyse zugeführt.

Im Sinne der vorliegenden Erfindung werden unter dem Begriff "Monophenol" sowohl Verbindungen wie Phenol selbst als auch haloginierte Phenole wie 2-Chlor-Phenol, 2-Bromphenol oder 2-Fluor-Phenol als auch Mischungen von zwei oder mehreren solcher Monophenole verstanden.

Die Waschphasen können nach Entfernung der Lösungsmittel- und Katalysatoranteile durch Strippen oder Extraktion gegebenenfalls wieder der Synthese zugeführt werden.

Ein Verfahren zur Herstellung von Diarylcarbonat und Verarbeitung mindestens eines Teils der dabei anfallenden alkalchloridhaltigen Abwassers in einer nachgeschalteten Alkalichlorid-Elektrolyse offenbart beispielsweise die WO2009071211A1.

Die Herstellung der Diarylcarbonate erfolgt gemäß dem Stand der Technik.

In einem Schritt (A) wird dazu ein Monophenol in Gegenwart eines Alkalihydroxids in einer wässrigen Phase gelöst und Phosgen in einem organischen Lösungsmittel gelöst.

In einem Schritt (B) erfolgt dann in einem Phasengrenzflächenverfahren die Umsetzung des Monophenols mit Phosgen unter Bildung eines in dem organischen Lösungsmittel gelösten Diarylcarbonats und eines Alkalihalogenids, das anschließend mit der wässrigen Phase abgetrennt wird. Das Alkalihydroxid ist bevorzugt Natriumhydroxid und das Alkalihalogenid ist bevorzugt NaCl. Als Diarylcarbonat bevorzugt ist Diphenylcarbonat. Optimierungen des Verfahrens durch Verbesserung der Durchmischung und Einhaltung eines engen Temperatur-und pH-Profils als auch Isolierung des Diarylcarbonates werden in EP 1 219 589 A1, EP 1 216 981 A2, EP 1 216 982 A2 und EP 784 048 A1 beschrieben.

Besonders geeignete Monophenole zur Herstellung der Diarylcarbonate in Schritt c) sind Phenole der Formel (VI) 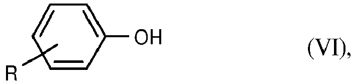 worin
- R: Wasserstoff, Halogen oder ein verzweigter oder unverzweigter C₁- bis C₉-Alkylrest oder Alkoxycarbonylrest ist.

Bevorzugt sind also Phenol, Alkylphenole wie Kresole, p-tert.-Butylphenol, p-Cumylphenol, p-n-Octylphenol, p-iso-Octylphenol, p-n-Nonylphenol und p-iso-Nonylphenol, oder Salicylsäure-methylester. Besonders bevorzugt ist Phenol.

Das eingesetzte Alkali zur Bildung des Phenolats kann eine Alkalilauge mit Hydroxiden aus der Reihe: Na-, K-, Li-Hydroxid sein, bevorzugt ist Natronlauge, und wird im neuen Verfahren vorzugsweise als 10 bis 55 Gew.%-ige Lösung eingesetzt.

Die Herstellung der Diarylcarbonate kann durch Katalysatoren, wie tertiäre Amine, N-Alkylpiperidine oder Oniumsalze beschleunigt werden. Bevorzugt werden Tributylamin, Triethylamin und N-Ethylpiperidin verwendet. Die Konzentrationen der Katalysatoren sind 0,0001 mol-% bis 0,1 mol-%, bevorzugt 0,01 mol-% bis 0,075 mol-%, bezogen auf das eingesetzte Monophenol.

Der eingesetzte Amin-Katalysator kann offenkettig oder zyklisch sein, besonders bevorzugt sind Triethylamin und N-Ethylpiperidin. Der Katalysator wird vorzugsweise als 1 bis 55 Gew.%-ige Lösung eingesetzt.

Unter Oniumsalzen werden hier Verbindungen wie NR₄X verstanden, wobei R ein Alkyl und/oder Arylrest und/oder ein H sein kann und X ein Anion ist.

Phosgen kann flüssig, gasförmig oder gelöst in einem inerten Lösungsmittel eingesetzt werden.

Gemäß dem Stand der Technik vorzugsweise verwendbare inerte Lösungsmittel für die Herstellung von Diarylcarbonaten, insbesondere für die Herstellung von Diphenylcarbonat, die für die Herstellung von Polycarbonaten verwendet werden können, sind Lösungsmittel auf der Grundlage von Toluol oder von Chlorkohlenwasserstoffen wie beispielsweise Dichlormethan, den verschiedenen Dichlorethanen oder Chlorpropanverbindungen, Chlorbenzol oder Chlortoluol, oder Gemischen aus zwei oder mehr dieser Chlorkohlenwasserstoffe und Toluol. Vorzugsweise wird ein inertes Lösungsmittel auf der Grundlage von Dichlormethan oder einem Gemisch aus Dichlormethan und Chlorbenzol eingesetzt. Im Rahmen der vorliegenden Erfindung werden solche inerten Lösungsmittel oder Gemische von inerten Lösungsmitteln zusammenfassend in der Einzahl "organisches Lösungsmittel" und in der Mehrzahl "organische Lösungsmittel" genannt.

Neben dem erhaltenen gewünschten Diarylcarbonat enthält das in Schritt (B) erhaltene Reaktionsgemisch, das bei der Umsetzung eines Monophenols mit Phosgen in einem organischem Lösungsmittel erhalten wird, im Wesentlichen noch nicht umgesetztes Monophenol, organisches Lösungsmittel, Alkalihalogenid und Wasser.

In einen Schritte (C) wird daher das Alkalihalogenid wie bereits weiter oben erklärt mit der wässrigen Phase abgetrennt und gegebenenfalls weiterverarbeitet.

In einem Schritt (D) erfolgt nun ein mehrstufiges Abtrennen sowohl des organischen Lösungsmittels, als auch des nicht umgesetzten Monophenols vom Diarylcarbonat.

Bei der Umsetzung des Monophenols mit Phosgen unter Bildung eines in dem organischen Lösungsmittel gelösten Diarylcarbonats in Schritt (B) im Phasengrenzflächenverfahren verbleibt neben dem Alkalihalogenid immer auch ein Teil nicht umgesetztes Monophenol in der abgetrennten wäßrigen Phase. Diese nicht umgesetzte, in der abgetrennten wässrigen Phase verbliebene Monophenol stört, da es zum einen die Rückgewinnung von Alkalihydroxid aus dem Alkalihalogenid erschwert, zum anderen die Belastung des Abwassers mit Aromaten erhöht, was die Aufbereitung des Abwassers vor einer Wiederverwendung oder auch einer Entsorgung erschwert. Ein hoher Anteil nicht umgesetzten Monophenols in der abgetrennten wäßrigen Phase ist daher sowohl aus Gründen der Wirtschaftlichkeit des Verfahrens als auch aus Gründen des Umweltschutzes unerwünscht.

Aufgabe der vorliegenden Erfindung ist es daher, ein Verfahren zur Herstellung eines Diarylcarbonats durch Reaktion eines Monophenols oder mehrerer Monophenole mit Phosgen in einem inerten Lösungsmittel in Gegenwart von Alkali und einer Stickstoffbase im sogenannten Phasengrenzflächenverfahren zur Verfügung zu stellen, bei dem - verglichen mit dem Stand der Technik - der Gehalt an Monophenol im Abwasser deutlich verringert ist.

Gelöst wird die Aufgabe durch ein Verfahren mit den Merkmalen des Hauptanspruchs. Insbesondere wird die Aufgabe gelöst durch
ein Verfahren zur Herstellung eines Diarylcarbonats umfassend folgende Schritte:
(A) Lösen eines Monophenols in Gegenwart eines Alkalihydroxids in einer wässrigen Phase gelöst und Phosgen in einem organischen Lösungsmittel;
(B) Umsetzung des Monophenols mit Phosgen unter Bildung eines in dem organischen Lösungsmittel gelösten Diarylcarbonats und eines Alkalihalogenids,
(C) Abtrennen des Alkalihalogenids mit der wässrigen Phase;
(D) mehrstufiges Abtrennen sowohl des organischen Lösungsmittels, als auch des nicht umgesetzten Monophenols vom Diarylcarbonat,
wobei der Gehalt an halognierten Monophenol in der Gesamtmenge des in Schritt (A) eingesetzten Monophenols kleiner als 10 ppm ist.

Wird dieser Grenzwert von kleiner als 10 ppm an halognierten Monophenol, beispielsweise 2-Chlor-Phenol oder 2-Fluor-Phenol, eingehalten, ist auch der Gehalt an Monophenol im Abwasser gering.

Bevorzugt ist der Gehalt an halognierten Monophenol in der Gesamtmenge des in Schritt (A) eingesetzten Monophenols kleiner als 5 ppm, besonders bevorzugt ist der Gehalt an halognierten Monophenol in der Gesamtmenge des in Schritt (A) eingesetzten Monophenols kleiner als 2 ppm.

Überraschenderweise wurde nämlich gefunden, dass der Anteil an Monophenol im Abwasser umso höher ist, je höher der Anteil an halogenierten Monophenol an der Gesamtmenge von Monophenol ist, das nicht bei der Reaktion mit Phosgen umgesetzt wurde. Dabei erhöht die Menge an halogeniertem Monophenol, das nicht bei der Reaktion mit Phosgen umgesetzt wurde, die Gesamtmenge an Monophenol im Abwasser überproportional. Daher muss darauf geachtet werden, dass der Anteil an halogenierten Monophenol an der Gesamtmenge von Monophenol, das nicht bei der Reaktion mit Phosgen umgesetzten wird, möglichst gering ist.

In einem Schritt (E) wird nun das Diarylcarbonat mit einem Diphenol oder mehreren Diphenolen zu einem Oligocarbonat und einem Monophenol umgesetzt, wobei das Oligocarbonat dann zu einem Polycarbonat weiter verarbeitet wird. Dazu wird das erhaltene Polycarbonat vom in Schritt (E) entstandenen Monophenol, von nicht umgesetztem Diarylcarbonat, von nicht umgesetztem Oligocarbonat und abgetrennt und anschließend weiter aufgereinigt, um das Polycarbonat als Endprodukt zu erhalten.

Das in diesem Schritt (E) gebildete Monophenol wird im Wesentlichen wieder Schritt (A) zugeführt. Das in diesem Schritt (E) nicht umgesetzte Diarylcarbonat wird im Wesentlichen abgetrennt und wieder Schritt (D) zugeführt.

Die Herstellung von aromatischen Oligo-/Polycarbonaten nach dem Schmelzeumesterungsverfahren ist literaturbekannt und beispielsweise in der Encyclopedia of Polymer Science, Vol. 10 (1969), Chemistry and Physics of Polycarbonates, Polymer Reviews, H. Schnell, Vol. 9, John Wiley and Sons, Inc. (1964) oder dem US-P 5 340 905 vorbeschrieben.

Die Erfindung soll durch die nachfolgend Beispiele näher erläutert werden, ohne dass die Erfindung auf diese Beispiele beschränkt werden soll:

### Beispiele

### Beispiel 1

In einer kontinuierlich betriebenen Laboranlage wurde bei 25 °C eine Natriumphenolatlösung (188,23 Gramm Phenol in 481,96 Gramm VE Wasser mit 81,21 Gramm Natronlauge) mit insgesamt 751,4 Gramm pro Stunde eingeleitet und mit 101,88 Gramm pro Stunde Phosgen in 846,69 Gramm eines Lösungsmittelgemischs (1:1 Gewichtsverhältnis Chlorbenzol : Methylenchlorid) gemischt. Das Gemisch wurde in eine Fink HMR040 Mischpumpe dosiert. Danach wurden 14,97 Gramm pro Stunde 32,09 gewichtsprozentige Natronlauge in Wasser hinzugefügt und in eine weitere Fink HMR040 Mischpumpe überführt. Das Reaktionsgemisch wurde in einen geflutet gefahrenen Rührreaktor mit N-Ethylpiperidin zur vollständigen Umsetzung zum Diphenylcarbonat gebracht. Die organische Phase wurde abgetrennt und mit 1 Gew.-%iger HCl-Lösung und anschließend mit VE Wasser gewaschen. Das Lösungsmittel wurde mittels Destillation entfernt.

Der Gehalt an Monophenolen im Abwasser betrug 41 ppm.

### Vergleichsbeispiel 1

Im Vergleichsbeispiel 1 wurde die gleiche Herstellmethode angewandt als in Beispiel 1, wobei das in Beispiel 1 eingesetzte Phenol im gleichen molaren Verhältnis ersetzt wurde durch 2-Chlor-Phenol. Der Gehalt an Monophenolen im Abwasser betrug 1310 ppm.

### Vergleichsbeispiel 2

Im Vergleichsbeispiel 2 wurde die gleiche Herstellmethode angewant als in Beispiel 1, wobei Phenol im gleichen molaren Verhältnis ersätzt wurde durch 2-Fluor-Phenol. Der Gehalt an Monophenolen im Abwasser betrug 2430 ppm
Den Beispielen kann eindeutig entnommen werden, dass der Anteil an Monophenol im Abwasser umso höher ist, je höher der Anteil an halogenierten Monophenol an der Gesamtmenge von Monophenol ist, das nicht bei der Reaktion mit Phosgen umgesetzt wurde. Dabei erhöht die Menge an halogeniertem Monophenol, das nicht bei der Reaktion mit Phosgen umgesetzt wurde, die Gesamtmenge an Monophenol im Abwasser überproportional. Umgekehrt kann den Beispielen eindeutig entnommen werden, dass der Anteil an Monophenol im Abwasser umso geringer ist, je geringer der Anteil an halogenierten Monophenol an der Gesamtmenge von Monophenol ist, das nicht bei der Reaktion mit Phosgen umgesetzt wurde.

## Patentansprüche

1. Verfahren zur Herstellung eines Diarylcarbonats umfassend folgende Schritte:
(A) Lösen eines Monophenols in Gegenwart eines Alkalihydroxids in einer wässrigen Phase gelöst und Phosgen in einem organischen Lösungsmittel;
(B) Umsetzung des Monophenols mit Phosgen unter Bildung eines in dem organischen Lösungsmittel gelösten Diarylcarbonats und eines Alkalihalogenids,
(C) Abtrennen des Alkalihalogenids mit der wässrigen Phase;
(D) mehrstufiges Abtrennen sowohl des organischen Lösungsmittels, als auch des nicht umgesetzten Monophenols vom Diarylcarbonat,
wobei der Gehalt an halognierten Monophenol in der Gesamtmenge des in Schritt (A) eingesetzten Monophenols kleiner als 10 ppm ist.

2. Verfahren nach Anspruch 1, wobei der Gehalt an halognierten Monophenol in der Gesamtmenge des in Schritt (A) eingesetzten Monophenols kleiner als 5 ppm ist.

3. Verfahren nach Anspruch 1, wobei der Gehalt an halognierten Monophenol in der Gesamtmenge des in Schritt (A) eingesetzten Monophenols kleiner als 2 ppm ist.

4. Verfahren zur Herstellung eines Polycarbonats umfassend folgende Schritte:
(A) Lösen eines Monophenols in Gegenwart eines Alkalihydroxids in einer wässrigen Phase gelöst und Phosgen in einem organischen Lösungsmittel;
(B) Umsetzung des Monophenols mit Phosgen unter Bildung eines in dem organischen Lösungsmittel gelösten Diarylcarbonats und eines Alkalihalogenids,
(C) Abtrennen des Alkalihalogenids mit der wässrigen Phase;
(D) mehrstufiges Abtrennen sowohl des organischen Lösungsmittels, als auch des nicht umgesetzten Monophenols vom Diarylcarbonat,
(E) Umsetzung des Diarylcarbonats mit einem Diphenol oder mehreren Diphenolen zu einem Oligocarbonat und einem Monophenol, wobei das Oligocarbonat dann zu einem Polycarbonat weiter verarbeitet wird,
wobei wobei der Gehalt an halognierten Monophenol in der Gesamtmenge des in Schritt (A) eingesetzten Monophenols kleiner als 10 ppm ist.

5. Verfahren nach Anspruch 4, wobei der Gehalt an halognierten Monophenol in der Gesamtmenge des in Schritt (A) eingesetzten Monophenols kleiner als 5 ppm ist.

6. Verfahren nach Anspruch 4, wobei der Gehalt an halognierten Monophenol in der Gesamtmenge des in Schritt (A) eingesetzten Monophenols kleiner als 2 ppm ist.
